## Europäisches Patentamt

## European Patent Office

## Office européen des brevets

(11) Veröffentlichungsnummer: **0 161 650**

**B1**

(12) **EUROPÄISCHE PATENTSCHRIFT**

(45) Veröffentlichungstag der Patentschrift:
04.02.87

(21) Anmeldenummer: 85105817.2

(22) Anmeldetag: 11.05.85

(51) Int. Cl.⁴: **C 07 C 87/60, C 07 C 85/24**

(54) **Verfahren zur Herstellung von 2,6-Dichlor-4-nitroanilin.**

(30) Priorität: 18.05.84 DE 3418495

(43) Veröffentlichungstag der Anmeldung:
21.11.85 Patentblatt 85/47

(45) Bekanntmachung des Hinweises auf die Patenterteilung:
04.02.87 Patentblatt 87/6

(84) Benannte Vertragsstaaten:
CH DE FR GB IT LI

(56) Entgegenhaltungen:
DD-A-159 425
DE-A-1 543 949
DE-B-2 648 054
GB-A-639 367
SU-A-863 590

PATENT ABSTRACTS OF JAPAN, unexamined
applications, Field C, Band 5, Nr. 94, 19. Juni 1981.
THE PATENT OFFICE JAPANESE GOVERNMENT,
Seite 11 C 59

(73) Patentinhaber: HOECHST AKTIENGESELLSCHAFT,
Postfach 80 03 20, D-6230 Frankfurt am Main 80
(DE)

(72) Erfinder: Arndt, Otto, Dr., Frankfurter Strasse 38,
D-6238 Hofheim am Taunus (DE)
Erfinder: Papenfuhs, Theodor, Dr., Heinrich-
Bleicher- Strasse 40, D-6000 Frankfurt am Main 50
(DE)

## Beschreibung

Gegenstand der Erfindung ist ein verbessertes Verfahren der an sich bekannten Herstellung von 2,6-Dichlor-4-nitroanilin aus 4-Nitroanilin durch Chlorieren mit unterchloriger Säure (HC10) in Wasser.

In der DB-PS 2 648 054 wird ein Verfahren zur Herstellung von Dichlornitroanilinen, beispielsweise 2,6-Dichlor-4-nitroanilin, wobei 4-Nitroanilin mit Hypochloriten in Gegenwart starker Säuren umgesetzt wird, beschrieben. Die starke Säure kann hierbei nach dort gemachten Angaben in dem weiten Konzentrationsbereich von 5 bis 400 Gew.-% (wasserfreie Säure), bezogen auf das Gewicht des zugesetzten Wassers, eingesetzt werden. Die in den Ausführungsbeispielen der genannten Patentschrift angegebenen Schmelzpunkte des erhaltenen 2,6-Dichlor-4-nitroanilins lassen erkennen, daß nach dem dort angewandten Verfahren Produkte minderer Qualität gewonnen werden. So bewegen sich die dort angegebenen Schmelzpunkte zwischen 174 und 182°C, während der höchste in der Literatur angegebene Schmelzpunkt 195°C beträgt (Atti della Reale Academia dei Lincei (Rendicontl (5) 22 I, 826)).

Auch gemäß den Angaben in SU 863 590 wird in einem großen Überschuß eines Gemisches von 30%iger Salzsäure (25 Mol) und 96%iger Schwefelsäure (14 Mol), jedoch bei etwas tieferer Temperatur gearbeitet. (Der Schmelzpunkt des hierbei erhaltenen 2,6-Dichlor-4-nitroanilins beträgt 190,5-191,5°C). Nachteilig bei diesem Verfahren ist die hohe Abwasserbelastung mit Salz-und Schwefelsäure.

In GB-PS 2 077 281 wird die Herstellung des inredestehenden Produkts bei relativ hohen Temperaturen (95-110°C) durch Chlorieren von 4-Nitroanilin mittels elementarem Chlor in 15-25%iger Salzsäure beschrieben. Das hierbei erhältliche Produkt ist von schlechter Qualität (Reingehalt etwa 82 bis maximal 90 %). Die Rohausbeuten sinken bei angewandten Salzsäurekonzentrationen von weniger als 14 % unter 75 %.

Es gibt auch Literaturhinweise, wonach bei der Chlorierung von 4-Nitroanlin mit Natriumhypochlorit extrem wenig Salzsäure eingesetzt wird, nämlich nur die Menge, die zur Neutralisation des bei der Chlorierung mit Natriumhypochlorit (NaOCl) freiwerdenden Natriumhydroxids (NaOH) nötig ist (Bulletin de la Société Chimique des France (4), 41, 197, 203). Auch hierbei ist eine schlechte Produktqualität zu erwarten, was daraus hervorgeht, daß sich die Autoren der genannten Literaturstelle auf eine Literaturstelle beziehen, nach welcher unter diesen Bedingungen Produkte schlechter Qualität zu erwarten sind (Körner, Jahresberichte über die Fortschritte der Chemie 1875, Seite 323, vgl. Beilstein, 12, 735, Syst.-Nr. 1671). Daß sich bei der Chlorierung von 4-Nitroanilin mit Natriumhypochlorit in sehr verdünnter wäßriger Salzsäure (z.B. 4,2%iger) stets verunreinigtes 2,6-Dichlor-5-nitroanilin bildet, geht auch aus J.Chem.Soc. 93, Seite 1773 (1908) hervor.

Die sich dabei bildenden harze sind vermutlich auf die Bildung von Stickstoff-Chlor-Verbindungen, wie beispielsweise N,N'-Tetrachlor-4-nitroanilin zurückzuführen; j. Che,. Soc. 123, 2791. Andere Verfahren verwenden organische Zusatzstoffe, teils als Katalysatoren (DDR 160111), teils als Verdünnungsmittel, oder organische Säure (DDR 160 111; J.Chem.Soc. 93, 1774; Austral. J.Chem. 12 (1959), 430).

Alle diese bekannten Verfahren sind sowohl in ökologischer und ökonomischer Hinsicht als auch hinsichlich der Qualität des Endproduktes mit erheblichen Nachteilen behaftet. Die ökologischen Mängel sind in der hohen Abwasserbelastung durch anorganische und organische Säuren, teilwiese auch durch Nitroaromaten (bei schlechten Ausbeuten) zu erblicken. Die ökonomischen Mängel liegen in dem zu leistenden technischen Aufwand zur Regeneration der organischen Zusatzstoffe und in der evtl. notwendigen Abwasseraufbereitung. Die Qualität des nach diesen bekannten Verfahren erhältlichen 2,6-Dichlor-4-nitroanilins ergibt sich aus den dort genannten Reingehalten und/oder Schmelzpunkten.

Es wurde nun gefunden, daß man 2,6-Dichlor-4-nitroanilin in hoher Reinheit (Reingehalt mindestens 96 %) und hoher Ausbeute (90 % der Theorie ohne die genannten Mängel, wie Abwasserbelastung und technischen Aufwand für Regeneration und Abwasseraufbereitung, durch Chlorieren von 4-Nitro-aninlin in Wasser mit Chlorbleichlauge herstellen kann, indem man 1 Mol 4-Nitroanilin in 3-6 Mol Salzsäure (HCl) oder Salpetersäure ($HNO_3$) in Form einer verdünnten, wäßrigen Säure in Gegenwart eines gegenüber den Reaktionsbedingungen stabilen Dispergiermittels chloriert, wobei man zunächst bei 5-10°C, dann bei 15-20°C chloriert und schließlich nach Umsetzung von 90-95 % des intermediär gebildeten 2-Chlor-4-nitroanlins zum 2,6-Dichlor-4-nitroanilin die Temperatur der wäßrigen Suspension ohne weiteren Zulauf von Chlorbleichlauge von 15-20° auf 70° erhöht, vorzugsweise unter erneuter Zugabe von Dispergiermittel, dann durch erneuten Zulauf von Chlorbleichlauge bei Temperaturen zwischen 20 und 70°C nachchloriert, anschließend die wäßrige Suspension auf pH 9,0 stellt, das gebildete 2,6-Dichlor-4-nitroanilin abfiltriert und mit verdünnter Mineralsäure wäscht.

Für die verfahrensgemäße Chlorierung des 4-Nitroanilins wird vorzugsweise Salzsäure (30-31 %ig) enigesetzt. Es ist aber auch möglich und besonders wirtschaftlich, die beim vorliegenden Verfahren anfallende Waschsäure (im Sinne eins recycling) durch Zusetzen zur vorgelegten Säure mitzuverwenden.

Bei Verwendung von Salzsäure bestimmt sich die Mindestmenge der einzusetzenden Säure

1) durch den Bedarf von 1 Äquivalent Salzsäure (HCl) bei der Bildung des 4-Nitroanilin-Hydrochlorids (Am. Soc. 52 (1930) 5115 und 5122) und von 2 Äquivalenten Salzsäure bei der Chlorierung des 4-Nitroanilins mit Natriumhypochlorit nach dem allgemeinen Reaktionsschema

HArH + 2 NaOCl → ClArCl + 2 NaOH

was insgesamt 3 Äquivalenten = 3 Mol) HCl pro Mol 4-Nitroanilin entspricht,

2) durch die Forderung, daß der Kontakt von 4-Nitroanilin bzw. 2-Chlor-4-nitroanilin mit unterchloriger Säure (HOCl) oberhalb pH 0,5 vermieden werden muß, wofür erfindungsgemäß zusätzlich zu den 3 Äquivalenten gemäß (1) 1-3 Äquivalente Salzsäure (HCl) ausreichen.

Werden beispielsweise nur 2 Äquivalente HCl eingesetzt, die gerade zur Neutralisation der 2 Äquivalente NaOH gemäß vorstehender Reaktionsgleichung ausreichen, die beim Umsatz von 1 Mol 4-Nitroanilin mit 2 Mol NaOCl freigesetzt werden, so erhält man ein Produkt in besonders schlechter Qualität und Ausbeute.

Insbesondere wurde auch gefunden, daß die in GB-PS 2 077 261 als nachteilig beschriebene Unterschreitung einer HCl-Konzentration von 14 % für die erfindungsgemäßen Verfahrensbedingungen keine Gültigkeit hat.

Durch die Nachbehandlung der Suspension bei 70°C (ohne weiteren Zulauf an Chlorbleichlauge) werden ca. 6 % 2-Chlor-4-nitroanilin freigelegt, die von ungelösten Feststoffpartikeln des 2,6-Dichlor-4-nitroanilins umhüllt waren.

Bei der Chlorierung in salzsaurem Medium ist es zweckmäßig, die Umsetzung in einem wäßrigen Medium durchzuführen, dessen HCl-Konzentration etwa 5 bis etwa 14 gewichtsprozentig ist.

Nachdem die in der Literatur beschriebenen Verfahren beträchtlich verschiedener Ausgestaltung nur unbefriedigende Ergebnisse liefern, (siehe weiter oben), muß es als überraschend erachtet werden, daß die erfindungsgemäße Chlorierung mit NaOCl gelingt, indem man eine Mindestmenge von 3 und eine Höchstmenge von 6 Mol Salzsäure oder Salpetersäure, außerdem ein Dispergiermittel einsetzt sowie eine thermische Nachbehandlung mit Nachchlorierung vornimmt. Entgegen der im Bulletin de la Soc. Chim. de France 1. c. aufgestellten Behauptung, daß keine Nebenreaktionen außer der Kernchlorierung ablaufen, bilden sich nach unseren Untersuchungen sehr wohl Nebenprodukte, die schon in Spuren die Qualität des Endprodukts wegen ihrer starken Farbintensität negativ beeinflussen. Ihre Entfernung bei der Filtrierung der alkalisch gestellten Suspension im Zuge des erfindungsgemäßen Verfahrens stellt einen deutlichen Fortschritt dar.

An das einzusetzende Dispergiermittel ist die Anforderung zu stellen, daß es gegenüber den Bedingungen der verfahrensgemäßen Chlorierung relativ stabil ist. Geeignete Dispergiermittel sind beispielsweise Verbindungen aus der Gruppe der Alkansulfonate (insbesondere sekundären Alkansulfonate), Aralkylsulfonate (Alkylbenzolsulfonate und Alkylnaphthalinsulfonate), primären Alkylsulfate (Fettalkoholsulfate, Fettalkylsulfate, Fettalkoholsulfonate), sekundären Alkylsulfate, Fettsäurekondensationsprodukte (Kondensate mit aminogruppenhaltigen Körpern, Kondensate mit oxygruppenhaltigen Körpern, Kondensate mit aromatischen Kohlenwasserstoffen), Polyglykoläther oder Pyridinbasen. Hinsichtlich der einzusetzenden Menge ist es zweckmäßig, etwa 5 - 45 Gewichtsteile Dispergiermittel pro Mol 4-Nitroanilin anzuwenden.

Nachstehend seien noch Einzelheiten bzw. bevorzugte Ausführungen des erfindungsgemäßen Verfahrens beschrieben:

Zur Vorbereitung der Chlorierung wird das 4-Nitroanilin in der 5-10-fachen Wassermenge unter Zusatz eines geeigneten Dispergiermittels, beispielsweise eines sekundären Alkansulfonats, verrührt. Zu der Suspension läßt man dann im Falle der Verwendung von Salzsäure mindestens 3 Äquivalente (3 Mol) 31%ige Salzsäure, vorzugsweise 5-6 Äquivalente (5-6 Mol), bezogen auf 1 Mol 4-Nitroanilin, zulaufen. Ein Zusatz von mehr als 6 Äquivalenten (6 Mol) HCl ist unnötig und würde nur zu einer erhöhten Belastung des Abwassers und verstärkter Emission von Chlor führen. Die Anwendung von 5-6 Äquivalenten (Mol) HCl bei einer Konzentration von ca. 13 %, bezogen auf die gesamte wässrige Lösung, wird deshalb bevorzugt, weil das 4-Nitroanilin dann in Lösung geht. Man kann auch Recycling-Säuren einsetzen, z.B. die zur Wäsche des 2,6-Dichlor-4-nitroanilins eingesetzte Mineralsäure, beispielsweise Salzsäure.

Unter Chlorbleichlauge versteht man beim erfindungsgemäßen Verfahren wäßrige Lösungen von NaOCl und NaCl, die durch Einleiten von Chlor in kalte wäßrige Natronlauge hergestellt werden können (eine 13,5 gewichtsprozentige Chlorbleichlauge enthält 166 g wirksames Chlor auf 1229 g = 1 Liter, entsprechend 2,34 Mol NaOCl auf 1 Liter) (Gmelins Handbuch der anorganischen Chemie 6 (1927), Seite 293; Ullmanns Encyklopädie der technischen Chemie 9, 4. Auflage (1975) Seite 544).

Vor der Chlorierung kühlt man die Suspension des 4-Nitroanilins bzw. die Lösung seines Hydrochlorids in der erfindungsgemäß eingesetzten Menge Salzsäure bzw. Salpetersäure auf 5-10°C ab.

Die Chlorierung verläuft in zwei Stufen:
1) Bildung von 2-Chlor-4-nitroanilin;
2) Bildung von 2,6-Dichlor-4-nitroanilin.

Beide Stufen erfordern etwas unterschiedliche Bedingungen hinsichtlich Temperatur und Zeitdauer. Die bevorzugten Temperaturen für die 1. Stufe liegen bei 5-10°C, für die 2. Stufe bei zunächst 15-20°C, dann bei 70°C. Die 2. Stufe ist hinsichtlich der Vollständigkeit des Umsatzes wesentlich kritischer als die 1. Stufe. Für die 1. Stufe werden 2-3 Stunden benötigt, hauptsächlich wegen des verstärkten Kühlbedarfs. Die 2. Stufe erfordert die erfindungsgemäße thermische Nachbehandlung, von 15-20°C ausgehend, bei 70°C in Gegenwart des vorzugsweise erneut zugesetzten Dispergiermittels mit der anschließenden Nachchlorierung bei 20 oder 70°C. Längere Zeiten für die 2. Stufe als 5-7 Stunden bei zunächst 15 bis 20°C und 2 Stunden bei der oberen Temperatur von 70°C sind wegen der dadurch bedingten Herabsetzung der Raumzeitausbeute unwirtschaftlich. Kürzere Zeiten als 1 Stunde bei der 2. Stufe führen zu unvollständigem Umsatz.

Bei optimaler Einhaltung der erfindungsgemäßen Bedingungen erreicht man schon mit Natriumhypochlorit-Mengen von nur wenig mehr als 2 Äquivalenten (z.B. 228 Mol.-%) einen vollständigen Umsatz des 4-Nitroanilins zum 2,6-Dichlor-4-nitroanilin. Bei ungünstigen Bedingungen können selbst wesentlich größere Überschüsse von Chlorbleichlauge nutzlos sein. Nach dünnschichtchromatographischer Überprüfung des Endes des Umsatzes wird evtl. noch vorhandenes restliches aktives Chlor mit einer äquivalenten Menge 40%iger Natriumhydrogensulfitlauge zerstört. Die hellgelbe Suspension wird bei 70°C filtriert. Es empfiehlt sich, hier die Möglichkeit einer Abtrennung von Spuren stark färbender Nebenkomponenten einzuschalten. Dazu wird die Suspension noch vor der Filtration mit 33%iger wäßriger Natronlauge auf pH 9,0 gestellt. An dieser Stelle kann sicherheitshalber nochmals 40%ige Natriumhydrogensulfitlauge zugegeben werden. Man erhält bei der Filtration dunkelbraune Mutterlaugen, während das abfiltrierte Produkt hell anfällt. Die Heißwäsche wird dann mit Wasser, ggf. unter weiterem Zusatz von Dispergiermittel, anschließend mit beispielsweise 2%iger Salzsäure und schließlich wieder mit Wasser ausgeführt. Die Filtrationen können auf offener Nutsche ausgeführt werden. Eine Belästigung durch Chlor ist nicht gegeben.

Man erhält das 2,6-Dichlor-4-nitroanilin in einer Ausbeute von 90 % der Theorie und mit einem Reingehalt von mindestens 97 % (gemäß high pressure liquid chromatography ("HPLC") vom Schmelzpunkt 187-191°C (gemäß Literaturangabe beträgt der Fp-Wert 192°C bei einem Reinheitsgehalt von 99,6 %). An Verunreinigung ist das 2-Chlor-4-nitroanilin (2,1 %) (HPLC) enthalten. Selbstverständlich läßt sich die Chlorierung auch auf der 1. Stufe unter Erhalt des 2-Chlor-4-nitroanilins stoppen (vgl. DRP 109 189 (1898)).

Der Vergleich mit den eingangs erwähnten literaturbekannten Verfahren zeigt deutlich die durch das erfindungsgemäße Verfahren gegebenen Fortschritte hinsichtlich der Einfachheit der Verfahrensweise und der Qualitätsverbesserung auf. 2,6-Dichlor-4-nitroanilin ist ein wichtiges Zwischenprodukt zur Herstellung von 3,5-Dichloranilin, welches ein Vorprodukt für Pflanzenschutzwirkstoffe ist. Außerdem ist es ein Zwischenprodukt zur Herstellung von Azofarbstoffen.

Die nachstehenden Beispiele dienen zur Erläuterung des erfindungsgemäßen Verfahrens, ohne es darauf zu beschränken. Teile bedeuten Gewichtsteile. Die Gewichtsteile des Dispergiermittels verstehen sich als standardisierte 60%ige wäßrige Lösungen.

**Beispiel 1**

(Wirkung der Menge anwesender Salzsäure) 140 Teile 4-Nitroanilin (1 Mol) in handelsüblicher technisch feuchter oder technisch trockener Form (Fp 147°C) werden in 1000 Teile Wasser, dem 5 Teile eines Dispergiermittels (sekundäres Alkansulfonat) zugesetzt sind, bei ca. 25°C unter üblichen Rührbedingungen eingetragen. Dann wird auf 5-10°C abgekühlt. Dazu läßt man 706 Teile 31%ige Salzsäure (6 Mol) zulaufen. Zu der Lösung tropft man unter Rühren und weiterer Außenkühlung bei 5-10°C 565 Teile 13,5%iger Chlorbleichlauge (entsprechend 1,07 Mol NaOCl = 1,07 Mol aktives Chlor) in 3 Stunden hinzu. Es kristallisiert das 2-Chlor-4-nitroanilin aus. Eine abfiltrierte Probe enthält nur ca. 1-2 % 4-Nitroanilin. Nach ggfs. erneuter Zugabe von 5 Teilen Dispergiermittel läßt man die Temperatur der Suspension auf 15-20°C ansteigen. Dann tropft man unter Rühren bei 15-20°C erneut 565 Teile 13,5 %iger Chlorbleichlauge (entsprechend 1,07 Mol NaOCl = 1,07 Mol aktives Chlor) in 5 Stunden hinzu.

Nach dünnschichtchromatographischer Überprüfung des Endes des Umsatzes wird noch vorhandenes restliches aktives Chlor mit einer äquivalenten Menge 40%iger wäßriger Natriumhydrogensulfitlauge zerstört, wozu in der Regel 15 Teile ausreichen. Anschließend wird in ca. 30 Minuten auf 60-65°C erwärmt. Die dünne, gelbe, feinkörnige Suspension wird mit 476 Teilen 33%iger Natronalauge von pH 0 auf pH 9,0 gestellt. Die nunmehr dünne, hellbraune Suspension wird nach nochmaligem Zusatz von 15 Teilen 40%iger Natriumhydrogensulfitlauge bei 70°C auf einer Nutsche abgesaugt. Die Mutterlauge ist dunkelbraun. Anschließend wird nacheinander gewaschen mit

1) 1000 Teilen Wasser von 70-75°C dem 2 5-Teile Dispergiermittel zugesetzt sind

2) 2000 Teilen einer 0,2 %igen Salzsäure bei 70-75°C und

3) 1000 Teilen Wasser von 70-75°C.

Man erhält nach dem Trocknen bei 60°C 191 Teile Trockenprodukt mit einem Reingehalt von 94,0 % 2,6-Dichlor-4-nitroanilin (HPLC) und einem Schmelzpunkt von 183-187°C, entsprechend einer Ausbeute von 86 % der Theorie als gelbes Pulver, dessen Lösungsfarbe in Dimethylformamid (100 mg/10 ml Dimethylformamid) hellbräunlichgelb ist. Der Gehalt an 2-Chlor-4-nitroanilin ist 5,5 %. Das Abwasser (ca. 6900-7000 Teile, pH 1,2) enthält ca. 16 Teile Nitroaromaten und kann nach entsprechender Vorbehandlung der biologischen Reinigung zugeführt werden.

**Beispiel 2**

(Wirkung der Menge anwesender Salzsäure)
Man verfährt wie in Beispiel 1 beschrieben, jedoch mit dem Unterschied, daß nur 353 Teile 31%ige Salzsäure (3 Mol) vorgelegt werden. Das 4-Nitroanilin geht nun nicht mehr in Lösung. Trotzdem erhält man nach dem Trocknen fast die gleiche Gewichtsmenge Trockenprodukt (193 Teile) wie gemäß Beispiel 1, jedoch mit einem niedrigeren Reingehalt von ca. 82 % 2,6-Dichlor-4-nitroanilin, einem Gehalt von 9 % 2-Chlor-4-nitroanilin und einem Schmelzpunkt von 176-180° C, entsprechend einer Ausbeute von 76 % der Theorie, als hellbraunes Pulver, dessen Lösungsfarbe in Dimethylformamid (100 mg/10 ml Dimethylformamid) braun ist.

Die Chlor-Emission am Ende der Chlorierung ist bei der Verfahrensdurchführung gemäß Beispiel 2 niedriger als gemäß Beispiel 1.

**Beispiel 3**

(Wirkung der Menge anwesender Salzsäure)
Man verfährt wie in Beispiel 1 beschrieben, jedoch mit dem Unterschied, daß nur 235 Teile 31%ige Salzsäure (2 Mol) vorgelegt werden. Man erhält eine ausgesprochen schlechte Ausbeute, nämlich nur 168 Teile Trockenprodukt mit einem Reingehalt von 47 % 2,6-Dichlor-4-nitroanilin (entsprechend einer Ausbeute von 38 % der Theorie) und einem Gehalt von 11 % 2-Chlor-4-nitroanilin sowie mit einem Schmelzpunkt von 150-165° C. Das Ergebnis ist auch wesentlich schlechter als das Ergebnis von Beispiel 2.

Man erkennt, daß der eigentliche Abfall von Qualität und Ausbeute dann eintritt, wenn weniger als 3 Mol Salzsäure vorgelegt werden.

**Beispiel 4**

(Ungünstige Wirkung der Anwendung einer zu großen Menge an Chlorbleichlauge im Zuge der zweiten Reaktionsstufe)
Man verfährt wie in Beispiel 1 beschrieben, jedoch mit dem Unterschied, daß nur 588 Teile 31%ige Salzsäure (5 Mol) vorgelegt werden und daß der 2. Anteil an zugesetzter Chlorbleichlauge 635 Teile beträgt.

Man erhält 187 Teile Trockenprodukt mit einem Reingehalt von 91 % 2,6-Dichlor-4-nitroanilin (entsprechend einer Ausbeute von 82 % der Theorie) und einem Gehalt von 5 % 2-Chlor-4-nitroanilin sowie einem Schmelzpunkt von 182-186° C, jedoch mit einer dunkleren Lösungsfarbe als bei der Verfahrensdurchführung gemäß Beispiel 1.

**Beispiel 5**

(Zusatz von Waschsäure zur vorgelegten Säure)
Man verfährt wie in Beispiel 4 beschrieben, jedoch mit dem Unterschied, daß zusätzlich zur vorgelegten Salzsäure Waschsäure (Salzsäure) eingesetzt wird, so daß insgesamt ca. 5,3 Mol HCl vorgelegt werden. Nach 2 maligem recycling erhält man 179 Teile Trockenprodukt mit einem Reingehalt von 96 % 2,6-Dichlor-4-nitroanilin (entsprechend einer Ausbeute von 83 % der Theorie) und einem Gehalt von 3 % 2-Chlor-4-nitroanilin.

**Beispiel 6**

(Auswirkung einer Abwesenheit von Dispergiermittel auf die Chlorierung)
Man verfährt wie in Beispiel 4 beschrieben, jedoch mit dem Unterschied, daß kein Dispergiermittel zugesetzt wird. Man erhält 183 Teile Trockenprodukt mit einem Reingehalt von nur 88 % 2,6-Dichlor-4-nitroanilin (entsprechend einer Ausbeute von nur 78 % der Theorie) und einem Gehalt von 6 % 2-Chlor-4-nitroanilin sowie einem Schmelzpunkt von nur 170-182° C. Die Lösungsfarbe ist etwas dunkler als bei der Verfahrensdurchführung gemäß Beispiel 4.

**Beispiel 7**

(Dieses Beispiel weist alle Merkmale des erfindungsgemäßen Verfahrens auf und stellt somit die optimale Ausführung dar)
Man verfährt wie in Beispiel 1 beschrieben, jedoch mit dem Unterschied, daß man nach Abreaktion von 1130 Teilen Chlorbleichlauge in der bei Beispiel 1 beschriebenen Form anschließend auf 70° C heizt und noch 10 Teile Dispergiermittel (sekundäres Alkansulfonat) zugibt (insgesamt also 22,5 Teile Dispergiermittel). Man läßt auf 20° C abkühlen und führt bei 20° C noch eine Nachchlorierung mit 70 Teilen Chlorbleichlauge aus. Man erhält 193 Teile Trockenprodukt mit einem Reingehalt von 96 % 2,6-Dichlor-4-nitroanilin (entsprechend einer Ausbeute von 90 % der Theorie) und einem Gehalt von 2,1 % 2-Chlor-4-nitroanilin sowie einem Schmelzpunkt von 187-191° C. Die Lösungsfarbe ist hellbräunlichgelb wie bei der Verfahrensdurchführung gemäß Beispiel 1.

**Beispiel 8**

(Ungünstige Auswirkung der Anwendung von etwas mehr Chlorbleichlauge)
Man verfährt wie in Beispiel 7 beschrieben, jedoch mit dem Unterschied, daß bei der Nachchlorierung 140 Teile Chlorbleichlauge eingesetzt werden. Man erhält nur 179 Teile Trockenprodukt vom Schmelzpunkt 186-189° C.

**Beispiel 9**

(Ungünstige Auswirkung der Anwendung von weniger Dispergiermittel)
Man verfährt wie in Beispiel 7 beschrieben, jedoch mit dem Unterschied, daß insgesamt nicht 22,5 sondern nur 10 Teile Dispergiermittel eingesetzt werden. Man erhält 187 Teile Trockenprodukt mit einem Schmelzpunkt von nur 180-185° C.

**Beispiel 10**

(Dieses Beispiel enthält alle Merkmale des erfindungsgemäßen Verfahrens)
Man verfährt wie in Beispiel 7 beschrieben, jedoch mit dem Unterschied, daß man die Nachchlorierung bei 70° C ausführt.
Man erhält 186 Teile Trockenprodukt mit einem Reingehalt von 97,5 % 2,6-Dichlor-4-nitroanilin (entsprechend einer Ausbeute von 88 % der Theorie) und einem Gehalt von 0,8 % 2-Chlor-4-nitroanilin sowie einem Schmelzpunkt von 187-191°C.
Die Lösungsfarbe ist hellorange und heller als bei der Verfahrensdurchführung gemäß Beispiel 7.

**Beispiel 11**

(Beschränkt nachteilige Auswirkung der Abwesenheit von Dispergiermittel bei Gegebensein aller anderen erfindungsgemäßen Verfahrensmerkmale)
Man verfährt wie in Beispiel 10 beschrieben, jedoch mit dem Unterschied, daß man kein Dispergiermittel verwendet. Man erhält 185 Teile Trockenprodukt mit einem Reingehalt von 96 % 2,6-Dichlor-4-nitroanilin (entsprechend einer Ausbeute von 86 % der Theorie) und einem Gehalt von 1,4 % 2-Chlor-4-nitroanilin sowie einem Schmelzpunkt von 183-188°C. Die Lösungsfarbe ist hellorange braun, etwas dunkler als bei der Verfahrensdurchführung gemäß Beispiel 10.

**Beispiel 12**

(Nachteilige Auswirkung einer zu schnellen Zugabe des zweiten Anteils von Chlorbleichlauge bei Fehlen einer Kühlung auf die Ausbeute)
Man verfährt wie in Beispiel 1 beschrieben, jedoch mit dem Unterschied, daß man den zweiten Anteil von 565 Teilen 13,5 %iger Chlorbleichlauge ohne weitere Außenkühlung in 15 Minuten zulaufen läßt, wobei die Temperatur auf 25° C ansteigt. Anschließend rührt man 1 Stunde bei 25° C nach und vervollständigt die Chlorierung durch die in den Beispielen 7 und 10 beschriebene thermische Nachbehandlung und Nachchlorierung bei 70° C. Man erhält 181 Teile Trockenprodukt mit einem Reingehalt von 97 % 2,6-Dichlor-4-nitroanilin (entsprechend einer Ausbeute von 85 % der Theorie) und einem Gehalt von 1,2 % 2-Chlor-4-nitroanilin sowie einem Schmelzpunkt von 186-188° C.

**Beispiel 13**

(Erhalt von ausschließlich Monochlorderivat des 4-Nitro-anilins bei Abbrechen der Reaktion nach der 1. Stufe)
Man verfährt wie in Beispiel 1 beschrieben, jedoch mit dem Unterschied, daß man nur den 1. Anteil Chlorbleichlauge zugibt. Man saugt das gebildete 2-Chlor-4-nitroanilin bei 10° C ab, wäscht mit 1000 Teilen Trinkwasser, 2000 Teilen 5%iger Sodalösung und nochmals mit 1000 Teilen Trinkwasser. Man erhält 167 Teile 2-Chlor-4-nitroanilin mit einem Schmelzpunkt von 94-101°C in einer Ausbeute von über 90 % der Theorie.

**Patentansprüche**

1. Verfahren zur Herstellung von 2,6-Dichlor-4-nitroanilin durch Chlorieren von 4-Nitroanilin mit Chlorbleichlauge in Wasser unter Verwendung von Säuren, dadurch gekennzeichnet, daß man 1 Mol 4-Nitroanilin in 3-6 Mol Salzsäure (HCl) oder Salpetersäure ($HNO_3$) in Form einer verdünnten, wäßrigen Säure in Gegenwart eines gegenüber den Reaktionsbedingungen stabilen Dispergiermittels chloriert, wobei man zunächst bei 5 bis 10° C, dann bei 15-20° C chloriert und schließlich nach Umsetzung von 90-95 % des intermediär gebildeten 2-Chlor-4-nitroanilins zum 2,6-Dichlor-4-nitroanilin die Temperatur der wäßrigen Suspension ohne weiteren Zulauf von Chlorbleichlauge von 15-20° C auf 70° C erhöht, anschließend durch erneuten Zulauf von Chlorbleichlauge bei Temperaturen zwischen 20° C und 70° C nachchloriert, die wäßrige Suspension auf pH 9,0 stellt, das gebildete 2,6-Dichlor-4-nitroanilin abfiltriert und mit verdünnter Mineralsäure wäscht.

2. Verfahren nach Anspruch 1, dadurch gekennzeichnet, daß man die Chlorierung in Gegenwart von 3 bis 6 Mol Salzsäure (HCl), bezogen auf eingesetzes 4-Nitroanilin, durchführt.

3. Verfahren nach Anspruch 1 oder 2, dadurch gekennzeichnet, daß man die Chlorierung in einem wäßrigen, salzsauren Medium durchführt, dessen Konzentraton an Salzsäure (HCl) etwa 5

bis etwa 14 gewichtsprozentig ist.

4. Verfahren nach Anspruch 1 oder 2, dadurch gekennzeichnet, daß man die Nachbehandlung durch Erhitzen der Suspension ohne Zulauf von Chlorbleichlauge unter erneuter Zugabe eines unter den Reaktionsbedingungen stabilen Dispergiermittels vornimmt.

## Claims

1. A process for the preparation of 2,6-dichloro-4-nitroaniline by chlorination of 4-nitroaniline with chlorine bleaching liquor in water using acids, which comprises chlorinating 1 mole of 4-nitroaniline in 3-6 moles of hydrochloric acid (HCl) or nitric acid (HNO₃) in the form of a dilute, aqueous acid in the presence of a dispersing agent which is stable under the reaction conditions, the chlorination initially being carried out at 5 to 10°C and then at 15-20°C and, finally, after 90-95% of the 2-chloro-4-nitroaniline intermediately formed has been converted into 2,6-dichloro-4-nitro-aniline, the temperature of the aqueous suspension being increased from -15-20°C to 70°C, without further addition of chlorine bleaching liquor, and then by post-chlorinating, by renewed addition of chlorine bleaching liquor, at temperatures between 20 and 70°C, bringing the pH of the aqueous suspension to 9.0 and filtering off the 2,6-dichloro-4-nitroaniline formed and washing it with dilute mineral acid.

2. The process as claimed in claim 1, wherein the chlorination is carried out in the presence of 3 to 6 moles of hydrochloric acid (HCl), based on the 4-nitro-aniline employed.

3. The process as claimed in claim 1 or 2, wherein the chlorinating is carried out in an aqueous hydrochloric acid medium, in which the concentration of hydrochloric acid (HCl) is about 5 to about 14 per cent by weight.

4. The process as claimed in claim 1 or 2, wherein the after-treatment is carried out by heating the suspension without the addition of chlorine bleaching liquor and with renewed addition of a dispersing agent which is stable under the reaction conditions.

## Revendications

1. Procédé de préparation de la dichloro-2,6 nitro-4 aniline par chloration de la nitro-4 aniline au moyen d'une lessive de blanchiment au chlore, dans de l'eau, avec emploi d'acides, procédé caractérisé en ce qu'on chlore 1 mole de nitro-4 aniline dans 3 à 6 moles d'acide chlorhydrique (HCl) ou d'acide nitrique (HNO₃) sous la forme d'un acide aqueux dilué, en présence d'un dispersant stable dans les conditions de la réaction, la chloration étant d'abord exécutée à 5-10°C, puis à 15-20°C, et, lorsque le taux de conversiom de la chloro-2 nitro-4 aniline intermédiairement formée en dichloro-2,6 nitro-4 aniline a atteint une valeur de 90 à 955, la température de la suspension aqueuse étant portée de 15-20°C à 70°C sans apport supplémentaire de lessive de blanchiment au chlore, après quoi on effectue une post-chloration à une température comprise entre 20 et 70°C en introduisant à nouveau de la lessive de blanchiment au chlore, on porte le pH de la suspension aqueuse à 9,0, on sépare par filtration la dichloro-2,6 nitro-4 aniline formée et on la lave avec un acide minéral dilué.

2. Procédé selon la revendication 1 caractérisé en ce qu'on effectue la chloration en présence de 3 à 6 moles d'acide chlorhydrique (HCl) par rapport à la nitro-4 aniline mise en jeu.

3. Procédé selon l'une des revendications 1 et 2 caractérisé en ce que la chloration est exécutée dans un milieu aqueux, acidifié par de l'acide chlorhydrique, dont la concentration en HCl est comprise entre environ 5 et environ 14% en poids.

4. Procédé selon l'une des revendications 1 et 2 caractérisé en ce que le traitement ultérieur par chauffage de la suspension sans apport de lessive de blanchiment au chlore est effectué avec une nouvelle introduction d'un dispersant stable dans les conditions réactionnelles.